# EUROPÄISCHE PATENTSCHRIFT

(11) **EP 1 690 851 B1**
(45) Veröffentlichungstag und Bekanntmachung des Hinweises auf die Patenterteilung: **28.04.2010**
(21) Anmeldenummer: 06002094.8
(22) Anmeldetag: 02.02.2006
(51) Int. Cl.: C07C 211/55, C07C 209/78, C07C 209/36, C07C 265/14, C07C 263/10

(54) **Verfahren zur Herstellung von Di-und Polyaminen der Diphenylmethanreihe**
Process for the preparation of di- and polyamines of the diphenylmethane series
Procédé de préparation de di- et polyamines de la série du diphénylmethane

(30) Priorität: 15.02.2005 DE 102005006692
(43) Veröffentlichungstag der Anmeldung: 16.08.2006
(73) Patentinhaber: Bayer MaterialScience AG, 51368 Leverkusen (DE)
(72) Erfinder: Grabowski, Stefan, Dr., B-9120 Melsele (BE); Dugal, Markus, Dr., 47906 Kempten (DE); Wolf, Aurel, Dr., 42489 Wülfrath (DE)

(56) Entgegenhaltungen:
- EP-A- 1 344 766
- EP-A- 1 524 260
- LAURA PRATI ET AL.: "Catalytic Hydrogen-Transfer Reduction of Organic Molecules with Methanol as Hydrogen Source" GAZZETTA CHIMICA ITALIANA, Bd. 122, 1992, Seiten 221-223, XP008064798

## Beschreibung

Die vorliegende Erfindung betrifft ein Verfahren zur Herstellung von Di- und Polyaminen der Diphenylmethanreihe (MDA) durch Umsetzung von Anilin mit Formaldehyd, bei dem das zur Herstellung benötigte Gemisch aus Anilin und Formaldehyd im für die MDA-Herstellung erforderlichen Mengenverhältnis simultan aus den Ausgangsverbindungen Nitrobenzol und Methanol hergestellt wird.

Anilin und Formaldehyd sind wichtige Zwischenprodukte unter anderem für die Polymerindustrie. Anilin und Formaldehyd werden zum Beispiel gemeinsam als Ausgangsstoffe zur Herstellung von Methylendiphenyldiamin und den entsprechenden Polyaminen (MDA) und Methylendiphenyldiisocyanat und den entsprechenden Polyisocyanaten (MDI), einem wichtigen Monomer für die Polyurethanherstellung, eingesetzt (EP 1344766). Für die Herstellung von Anilin und Formalin existieren jeweils eine Reihe von Verfahren, die zum Teil technisch umgesetzt wurden. Die industrielle Herstellung von Anilin erfolgt derzeit durch die katalytische Gasphasenhydrierung von Nitrobenzol mit Wasserstoff in adiabater (Hydrocarbon Process **59** (Nov. 1979) no. 11, 136; US-A-3,636,152) oder isothermer Fahrweise (US-A-4,265,834) unter Verwendung von Cu- oder Pd- Katalysatoren. Von untergeordneter Bedeutung sind die Reduktion von Nitrobenzol mit Eisen (Bechamp-Verfahren, Winnacker-Küchler Chemische Technologie, 3rd ed., Vol. 4, pp. 170 - 171) und die heterogen katalysierte Gasphasen-Ammonolyse von Phenol (Halcon-Verfahren, US-A-3,272,865).

Die Herstellung von Formaldehyd im technischen Maßstab erfolgt derzeit im wesentlichen durch Silber-katalysierte Dehydrierverfahren (DE-A-2 322 757, US-A-2,519,788) und das sogenannte Formox-Vefahren (GB-A-1 080 508).

Bei den Silber-katalysierten Verfahren wird Methanol durch Luft bei > 600°C an einem Silberkatalysator unter Bildung von Formaldehyd und Wasserstoff dehydriert, wobei der Wasserstoff im weiteren Reaktionsverlauf bzw. in nachgeschalteten Reaktionsstufen mit Luftsauerstoff zwecks Energieerzeugung zu Wasser umgesetzt wird. Das Formox-Verfahren beinhaltet eine zweistufige Oxidation von Methanol zu Formaldehyd und Wasser (Oxidations-Reduktionszyklus des Katalysators), die bei niedrigeren Temperaturen im Bereich 270-300°C in der Regel unter Einsatz von Molybdän-Eisenkatalysatoren abläuft.

Aus der Anwendung der erwähnten Verfahren folgt zwangsläufig, dass die Produkte Anilin und Formaldehyd in getrennten Anlagen unabhängig voneinander hergestellt und aufgearbeitet werden müssen. Für die Herstellung von Anilin, insbesondere durch die technisch maßgeblichen Hydrierverfahren, muss dabei zudem Wasserstoff als kostenintensives Reduktionsmittel eingesetzt werden.

Für die Herstellung von Methylendiphenyldüsocyanat und den entsprechenden Polyisocyanaten (MDI) wird MDA mit Phosgen umgesetzt. Das für die Phosgenierung benötigte Phosgen wird dabei üblicherweise durch einen technischen Prozess erzeugt, bei dem Kohlenmonoxid und Chlor über Aktivkohle geleitet und dabei zur Reaktion gebracht wird. Die Reaktion ist stark exotherm. Üblicherweise wird ein gekühlter Rohrbündelreaktor verwendet, dessen Röhren mit granulierter Aktivkohle gepackt sind. Die Temperatur des Aktivkohlebetts in der Reaktionszone liegt bei ca. 400°C und sinkt durch die Kühlung entlang der Röhren auf 40 - 150°C ab. Es ist auch möglich, die Reaktion in zwei Stufen durchzuführen, wobei die erste bei hoher Temperatur (200 - 400°C), die zweite bei tieferer Temperatur (40 - 150°C) durchgeführt wird. In den meisten Anwendungen ist ein möglichst geringer Restchlorgehalt wünschenswert. Deshalb wird üblicherweise Kohlenmonoxid im stöchiometrischen Überschuss eingesetzt. Die Reaktion wird unter Atmosphärendruck durchgeführt. Das gebildete, gasförmige Phosgen wird in einem weiteren Prozessschritt in Lösemittel absorbiert. Diese Lösung wird anschließend in der Herstellung der Isocyanate durch Umsetzung mit Di- und/oder Polyaminen verwendet.

Für die Herstellung von MDA durch säurekatalysierte Umsetzung von Anilin und Formaldehyd wäre es von Vorteil, Anilin und Formaldehyd simultan in einem Verfahren herzustellen, um weniger Anlagenteile zu benötigen und das Verfahren zu vereinfachen. Weiterhin wäre es im Hinblick auf die Wirtschaftlichkeit und die Sicherheit des Verfahrens von Vorteil, bei der Nitrobenzol-Reduktion zu Anilin den Hydrierwasserstoff durch eine günstigere und besser handhabbare Wasserstoffquelle zu ersetzen, die zudem Wasserstoff unter Entstehung eines Wertstoffes überträgt.

Es wurde nun gefunden, dass die oben beschriebene Aufgabenstellung durch die katalytische Transferhydrierung von Nitrobenzol und Methanol nach Gleichung (I) erreicht werden kann.

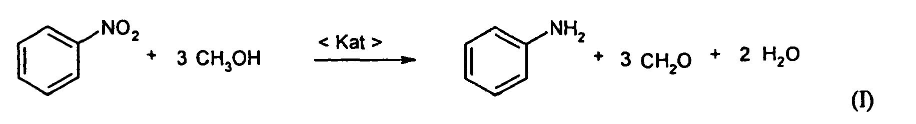

Ein gemäß Gleichung (I) hergestelltes Gemisch weist jedoch ein molares Verhältnis von Anilin zu Formaldehyd von 1:3 auf. Für eine technische Synthese von MDA werden jedoch üblicherweise Gemische mit einem molaren Verhältnis von Anilin zu Formaldehyd von 1,5:1 bis 10:1 (entsprechend 1:0,1 bis 1: 0,67) eingesetzt. Üblicherweise wird Anilin dabei nicht vollständig umgesetzt, so dass Teile des eingesetzten Anilins nach der Reaktion zurückgeführt werden. Dieser zurückgeführte Teil des Anilins wird üblicherweise mit frischem Anilin versetzt und erneut mit Formaldehyd zu MDA umgesetzt. Für die frischen Anteile an Anilin und das in die Reaktion eingesetzte Formaldehyd wird technisch ein molares Verhältnis von 0,9:1 bis 2,4:1 (entsprechend 1:1,1 bis 1:0,42) eingestellt.

Produktgemische, die nach Gleichung (I) erhalten werden, müssten also mit einer erheblichen Zusatzmenge an Anilin versetzt werden, das nach einem konventionellen Verfahren hergestellt wurde, damit diese Gemische für eine technische MDA-Synthese geeignet sind. Damit sinkt die Wirtschaftlichkeit des Verfahrens jedoch erheblich, da zusätzlich zur Transferhydrierung nach Gleichung (1) eine konventionelle Nitrobenzol-Hydrierung erforderlich bleibt.

Die Aufgabe der vorliegenden Erfindung bestand daher darin, ein einfaches und wirtschaftliches Verfahren zur Herstellung von MDA zur Verfügung zu stellen, bei dem auf den Einsatz von Anilin, das durch Hydrierung von Nitrobenzol hergestellt wurde, verzichtet werden kann.

Die Erfindung betrifft ein Verfahren zur Herstellung von Di- und Polyaminen der Diphenylmethanreihe, bei dem man
a) Nitrobenzol und Methanol in Gegenwart eines Katalysators zu Anilin, Formaldehyd und Kohlenmonoxid umsetzt, und anschließend
b) das in Schritt a) hergestellte Anilin und Formaldehyd in Gegenwart eines sauren Katalysators zu Di- und Polyaminen der Diphenylmethanreihe umsetzt, und
c) das in Schritt a) hergestellte Kohlenmonoxid mit Chlor zu Phosgen umsetzt.

In Schritt a) können dabei, neben weiteren Reaktionen, insbesondere folgende Reaktionen ablaufen:

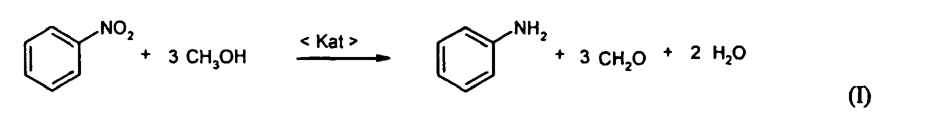

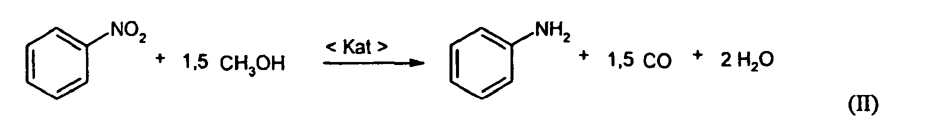

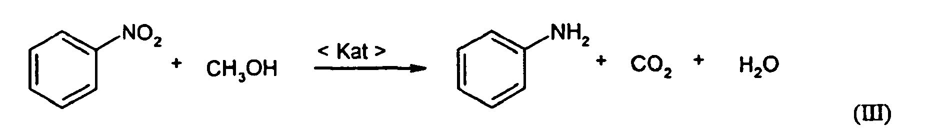

Die in den Gleichungen (I) bis (III) gezeigten Reaktionen können dabei direkt in der gezeigten Weise ablaufen. Es ist aber auch möglich, dass beispielsweise die Bildung von CO gemäß Gleichung (II) über Formaldehyd als Zwischenstufe abläuft bzw. die Bildung von CO₂ über Formaldehyd und/oder CO als Zwischenstufen abläuft. Ebenso ist es möglich, dass neben CO andere Zwischen- oder Folgeprodukte entstehen, bei denen der Kohlenstoff in der Oxidationsstufe -2 vorliegt, z.B. Ameisensäure und ihre organischen und/oder anorganischen Derivate. Des weiteren ist es möglich dass neben CO₂ andere Zwischen- oder Folgeprodukte entstehen bei denen der Kohlenstoff in der Oxidationsstufe -4 vorliegt, z.B. Kohlensäure und ihre organischen und/oder anorganischen Derivate wie z.B. Carbonate, Carbamate, Harnstoffe, Isocyanate.

Die Herstellung von Anilin durch katalytische Transferreduktion von Nitrobenzol mit Methanol ist von Rossi et al. (Gaz. Chim. It., 122, 1992, 221-223) beschrieben. Dabei wird ein Cu-Katalysator bei Temperaturen von 180° verwendet. Als Reaktionsprodukt wird bei einem Umsatz von 58% lediglich Anilin angegeben. Rossi et al. diskutieren jedoch die theoretische Möglichkeit, dass Formaldehyd, Methylformiat, CO und CO₂ als Nebenprodukte der Reaktion entstehen könnten. Ein experimenteller Nachweis dafür wird aber nicht gegeben. Die Möglichkeit, dass durch die Transferreduktion von Nitrobenzol mit Methanol ein Reaktionsproduktgemisch enthaltend Anilin und Formaldehyd mit einer Zusammensetzung erhalten werden können, die einen direkten Einsatz in der Herstellung von MDA erlauben, wird in Gaz. Chim. It., 122, **1992**, 221-223 nicht in Betracht gezogen.

Geeignete Katalysatoren für die Transferreduktion von Nitrobenzol in Schritt a) sind zum Beispiel anorganische, im Reaktionsmedium nicht lösliche (heterogene) Katalysatoren oder lösliche (homogene) Metallkomplexe oder Salze, wobei diese Katalysatoren ein oder mehrere Metalle als katalytisch wirksame Komponenten in elementarer oder gebundener Form enthalten. Geeignete Metalle sind beispielsweise Pd, Pt, Rh, Ir, Ru, Fe, Co, Ni, Cu, A1, Mg, Zr, Zn, V, Cr, Mo, W, Pb, Lanthanoide. Bevorzugt werden Pd, Pt, Ir, Ru, Cu, Ni oder Fe-haltige Katalysatoren eingesetzt.

Die Umsetzung von Nitrobenzol mit Methanol in Schritt a) erfolgt vorzugsweise in Gegenwart von Hilfsstoffen. Geeignete Hilfsstoffe sind beispielsweise basische anorganische oder organische Verbindungen die löslich oder unlöslich im Reaktionsmedium sind oder Lösungsmittel. Geeignete Basen sind z.B. Hydroxide wie NaOH, KOH oder NH₄OH, Carbonate wie Na₂CO₃ oder K₂CO₃, Hydrogencarbonate wie NaHCO₃, Amine wie Triethylamin oder Anilin oder unlösliche basische Festsstoffe wie z.B. Hydrotalcite, Al₂O₃, MgO. Unlösliche basische Feststoffe können gegebenenfalls gleichzeitig als Base und als Trägermaterial für den Katalysator dienen. Bevorzugte Basen sind NaOH, KOH, Hydrotalcite oder Aluminiumoxid.

Geeignete Lösungsmittel sind beispielsweise Wasser, Alkohole, organische Amine und/oder Nitroverbindungen. Bevorzugte Lösungsmittel sind die an der Reaktion beteiligten Komponenten Methanol, Nitrobenzol, Wasser und Anilin.

Die Reaktion kann generell in der Gas- und/oder in der Flüssigphase durchgeführt werden. Geeignete Reaktionstemperaturen liegen üblicherweise im Bereich von 20°C - 500°C, bevorzugt im Bereich von 50°C - 300°C. Der absolute Reaktionsdruck liegt üblicherweise im Bereich von 0,1 bar bis 300 bar, bevorzugt im Bereich von 1 bar bis 100 bar. Die Konzentrationen und Konzentrationsverhältnisse der Ausgangsverbindungen sowie der Hilfsstoffe können im Prinzip frei gewählt werden. Je nach Wahl der Reaktionsbedingungen kann in der Reaktion Teil- oder Vollumsatz bezogen auf Methanol oder Nitrobenzol erreicht werden.

Das nach dem erfindungsgemäßen Verfahren hergestellte Produktgemisch weist bevorzugt ein molares Verhältnis von Anilin zu Formaldehyd von 0,9:1 bis 2,4:1 und ein molares Verhältnis von Anilin zu CO von 10:1 bis 0,5:1 auf.

Bevorzugt werden Methanol und Nitrobenzol in einem molaren Verhältnis von 3:1 bis 10:1 in die Reaktion eingesetzt. Das nach der Reaktion erhaltene Produktgemisch besteht nach Abkühlung auf Umgebungstemperatur (25°C) und Entspannung auf Atmosphärendruck aus einer Gasphase, die Kohlenmonoxid, Kohlendioxid und ggf. weitere gasförmige Komponenten, wie z.B. Wasserstoff enthält, und einer flüssigen Phase, die Anilin und Formaldehyd, ggf. überschüssiges Methanol und ggf. überschüssiges Nitrobenzol, sowie ggf. gebildete Nebenprodukte wie z.B. N-Methylanilin, Toluidine, N-Formylanilin, N-Phenylmethylcarbamat, Aminobenzylaniline u.a. enthält.

Die Reaktionsprodukte Anilin und Formaldehyd und die gegebenenfalls auftretenden Nebenkomponenten sowie nicht umgesetztes Nitrobenzol und Methanol sowie die eingesetzten Hilfsstoffe können teilweise oder vollständig aus der Reaktionsmischung abgetrennt und gegebenenfalls zu den Reinverbindungen aufgearbeitet werden. Auf diese Weise erhaltenes Anilin und / oder Formaldehyd kann neben der MDA-Herstellung prinzipiell auch anderen Anwendungen zugeführt werden. Gegebenenfalls isolierte Nebenverbindungen wie beispielsweise CO₂ stehen ebenfalls prinzipiell für andere Anwendungen zur Verfügung. Nicht umgesetztes Nitrobenzol und / oder Methanol werden vorzugsweise in den Reaktionskreislauf zurückgeführt. CO wird ebenfalls aus der Reaktionsmischung, beispielsweise durch Gasabtrennung, entfernt und gegebenenfalls vor der Umsetzung mit Chlor zu Phosgen in Schritt c) beispielsweise durch Tieftemperatur-Kondensation und Fraktionierung, einen Adsorptions-/Desorptionsprozess oder ähnlichen Verfahren gereinigt.

Alternativ können die Reaktionsprodukte Anilin und Formaldehyd zusammen mit den nicht umgesetzten Ausgangsverbindungen Nitrobenzol und Methanol sowie gegebenenfalls den Hilfsstoffen vollständig oder teilweise in der Reaktionsmischung belassen werden und in Schritt b) direkt zu MDA umgesetzt werden.

In Schritt b) werden das in Schritt a) hergestellte Anilin und Formaldehyd gegebenenfalls nach vorheriger Aufreinigung weiter umgesetzt zu MDA. Dazu wird dem Gemisch enthaltend Anilin und Formaldehyd ein saurer Katalysator zugesetzt.

Geeignete saure Katalysatoren sind starke organische oder anorganische Säuren, beispielsweise Salzsäure, Schwefelsäure, Phosphorsäure, Methansulfonsäure oder feste Säuren wie z.B. Zeolithe. Bevorzugt wird Salzsäure eingesetzt.

Das Reaktionsgemisch wird im Regelfall nach der Vermischung einer Vorreaktion im Temperaturbereich zwischen 20°C und 100°C, vorzugsweise im Temperaturbereich von 30°C bis 80°C unterzogen und anschließend in Stufen oder kontinuierlich und ggf. unter Überdruck auf eine Temperatur von 100°C bis 250°C bevorzugt auf 100°C bis 180°C, besonders bevorzugt auf eine Temperatur von 100°C bis 160°C gebracht. Auf die Vorreaktion kann aber auch verzichtet werden.

Das danach erhaltene Reaktionsgemisch wird anschließend vorzugsweise mit einer Base neutralisiert und in einem Scheidebehälter die wässrige und die organische getrennt. Das MDA ist dann in der organischen Phase enthalten.

Erfindungsgemäß wird in Schritt a) neben dem Gemisch aus Anilin und Formaldehyd, das in einem für die MDA-Herstellung geeigneten molaren Verhältnis erhalten wird, auch CO gebildet. Das so erzeugte CO wird anschließend in Schritt c) mit Chlor zu Phosgen umgesetzt. Ein geeignetes Verfahren zur Herstellung von Phosgen aus CO und Chlor ist beispielsweise in EP-A-134 506 beschrieben. Dabei wird CO und Chlor in Rohrbündelreaktoren an Aktivkohle-Katalysatoren bei Temperaturen von unter 100°C am Reaktoraustritt umgesetzt. In einer bevorzugten Ausführungsform wird das so hergestellte Phosgen für die Phosgenierung des in Schritt b) erzeugten MDA zu MDI eingesetzt.

Die Erfindung betrifft weiterhin ein Verfahren zur Herstellung von Di- und Polyisocyanaten der Diphenylmethanreihe, bei dem man
a) Nitrobenzol und Methanol in Gegenwart eines Katalysators zu Anilin, Formaldehyd und Kohlenmonoxid umsetzt, und anschließend
b) das in Schritt a) hergestellte Anilin und Formaldehyd in Gegenwart eines sauren Katalysators zu Di- und Polyaminen der Diphenylmethanreihe umsetzt, und
c) das in Schritt a) hergestellte Kohlenmonoxid mit Chlor zu Phosgen umsetzt, und
d) die in Schritt b) hergestellten Di- und Polyamine der Diphenylmethanreihe durch Phosgenierung zu den Di- und Polyisocyanaten der Diphenylmethanreihe umsetzt.

Dazu wird zusätzlich zu dem bereits beschriebenen Verfahren zur Herstellung von Di- und Polyaminen der Diphenylmethanreihe (Schritte a) bis c)) das in Schritt b) hergestellte MDA anschließend nach den bekannten Methoden mit Phosgen in einem inerten organischen Lösungsmittel zu den entsprechenden Isocyanaten umgesetzt. Das Molverhältnis von Roh-MDA aus Schritt b) zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird zweckmäßigerweise so bemessen, dass die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist. Nach beendeter Phosgenierung wird der Reaktionsmischung das überschüssige Phosgen, das inerte organische Lösungsmittel oder Mischungen davon durch Destillation abgetrennt.

### Beispiele:

### Beispiel 1a ( erfindungsgemäß)

### Katalysator: 5 Gew.-% Pd auf Aluminiumoxid

In einem Rührgefäß aus VA-Stahl werden 1,233 g Nitrobenzol, 3,232 g Methanol und 0,119 g Palladium-Katalysator (5 Gew.-% Pd auf basischem Aluminiumoxid) vorgelegt. Das Gefäß wird verschlossen und in einem Ölbad unter Magnetrührung bei 195°C 150 min lang unter Eigendruck zur Reaktion gebracht. Dann wird das geschlossene Gefäß auf Raumtemperatur abgekühlt, eine Probe aus dem Gasraum entnommen und anschließend auf Umgebungsdruck entspannt. Der Katalysator wird abzentrifugiert und die überstehende Lösung abgetrennt.

Die Analytik der Gasprobe sowie der flüssigen Reaktionsmischung ergibt, dass sich folgende Produkte gebildet haben:
- Anilin:: 0,80 mmol
- Formaldehyd:: 0,532 mmol
- CO:: 0,53 mmol
- CO₂:: 0,19 mmol

Das molare Verhältnis von Anilin : Formaldehyd beträgt demnach 1,50, das molare Verhältnis von Anilin zu CO beträgt 1,51.

In entsprechender Weise werden die Beispiele 1 b bis 1 1 durchgeführt, deren Ergebnisse in **Tabelle 1** zusammengefasst sind. Bei Verwendung von Mischmetall - Katalysatoren sind dabei zusätzlich die Verhältnisse der Gew.-Anteile der Metallkomponenten angegeben.

**Tabelle 1**

| | Kat | | Einwaagen [g] | | | Reaktions-bedingungen | | Zusammensetzung Produktgemisch [mmol] | | | | | Anilin: Formaldehyd | Anilin : CO |
|---|---|---|---|---|---|---|---|---|---|---|---|---|---|---|
| | Metall (5 Gew.-%) | Träger | Kat | Nitro-benzol | Methanol | Zeit [min] | Temp. [°C] | Anilin | Nitro-benzol | Formaldehyd | CO | CO2 | | |
| | | | | | | | | | | | | | | |
| a | Pd | Aluminium -oxid | 0,119 | 1,23 | 3,23 | 150 | 195 | 0,80 | 8,69 | 0,53 | 0,53 | 0,19 | 1,51 | 1,51 |
| b | Pd/Ni 1:1 | Aluminium -oxid | 0,160 | 1,23 | 3,22 | 140 | 190 | 0,88 | 9,12 | 0,58 | 0,36 | 0,10 | 1,52 | 2,44 |
| c | Pd | Aluminium -oxid | 0,448 | 1,24 | 3,22 | 140 | 187 | 2,01 | 7,59 | 1,05 | 1,08 | 0,55 | 1,91 | 1,86 |
| d | Pd | Aluminium -oxid | 0,142 | 1,24 | 2,69 | 162 | 190 | 1,04 | 9,03 | 0,68 | 0,54 | 0,12 | 1,53 | 1,93 |
| e | Pd/Ir 96:4 | Aluminium -oxid | 0,298 | 1,24 | 2,71 | 159 | 190 | 1,36 | 8,06 | 0,71 | 0,89 | 0,50 | 1,92 | 1,53 |
| f | Pd/Ir 3:1 | Hydrotalcit | 0,253 | 1,24 | 3,22 | 130 | 190 | 3,16 | 6,33 | 1,32 | 0,38 | 1,26 | 2,39 | 8,32 |
| g | Pd/Ir 1:1 | Aluminium -oxid | 0,579 | 1,24 | 3,26 | 140 | 185 | 1,06 | 8,70 | 0,60 | 0,39 | 0,60 | 1,77 | 2,72 |
| h | Pd/Ni 3:1 | Aluminium -oxid | 0,372 | 1,23 | 1,61 | 170 | 170 | 1,06 | 8,89 | 0,59 | 0,28 | 0,27 | 1,80 | 3,79 |
| i | Pd/Ru 6:4 | Aluminium -oxid | 0,330 | 1,23 | 1,62 | 130 | 180 | 0,96 | 8,96 | 0,45 | 0,23 | 0,16 | 2,13 | 4,17 |
| k | Pd/Ni 9:1 | Aluminium -oxid | 0,415 | 1,23 | 1,62 | 120 | 180 | 1,25 | 8,29 | 0,62 | 0,17 | 0,12 | 2,02 | 7,35 |
| l | Pd/Ni 1:9 | Aluminium -oxid | 0,259 | 1,23 | 3,22 | 180 | 200 | 1,16 | 8,56 | 0,56 | 0,58 | 0,22 | 2,07 | 2,00 |
| m | Pd/Cr/Ni 1:2:2,5 | Aluminium -oxid | 0,261 | 1,23 | 6,41 | 60 | 195 | 0,996 | 8,851 | 0,536 | 1,21 | 0,044 | 1,86 | 0,82 |

Ein nach dem erfindungsgemäßen Verfahren mittels katalytischer Transferhydrierung hergestelltes Reaktionsgemisch (804,2 g) der folgenden Zusammensetzung

| Anilin (Gew.-%) | Nitrobenzol (Gew.-%) | Formaldehyd (Gew.-%) | Methanol (Gew.-%) | Wasser (Gew.-%) |
|---|---|---|---|---|
| 12,4 | 45,9 | 1,8 | 35,8 | 4,1 |

| | | | | |
|---|---|---|---|---|
| wird wie folgt weiterverarbeitet: | | | | |

Aus diesem Gemisch werden Methanol und Wasser durch Destillation abgetrennt. Der verbleibende Destillationssumpf (479,0 g) wird in einer Glas-Rührapparatur unter Stickstoffüberlagerung auf 35°C temperiert. Bei dieser Temperatur werden 11,4 g 32,7%ige Salzsäure innerhalb 5 min zugetropft. Dabei wird die Temperatur durch ein Eisbad bei 35°C gehalten und bei dieser Temperatur 30 min weiter gerührt. Dann wird auf 60°C aufgeheizt und weitere 30 min bei dieser Temperatur gerührt. Dann wird der Reaktionsansatz zum Sieden erhitzt und 10 h unter Rückfluss bei der Siedetemperatur (103°C) gehalten. Dann werden 14,9 g 32%ige Natronlauge und 100 g destilliertes Wasser zugegeben und das entstehende zweiphasige Gemisch 15 min kräftig durchmischt. Anschließend werden die Phasen getrennt und die organische Phase noch zweimal mit je 100 g dest. Wasser extrahiert. Aus der organischen Phase werden überschüssiges Anilin sowie Wasser und Nitrobenzol im Vakuum (0,1 mbar) abdestilliert. Die Sumpftemperatur am Ende der Destillation beträgt 215°C. Das hergestellte MDA (= Destillationssumpf, 75,0 g) hat folgende Zusammensetzung:

| | Gew.-% |
|---|---|
| Anilin | - |
| Nitrobenzol | - |
| 4,4'-MDA | 38,6 |
| 2,4'-MDA | 4,46 |
| 2,2'-MDA | 0,3 |
| N-Methyl-MDA | 0,65 |
| 3-Kern-MDA | 19,9 |
| 4-Kem-MDA | 11,2 |
| höhere MDA-Oligomere | 15,3 |
| weitere Nebenkomponenten und Unbekannte | 9,59 |

## Patentansprüche

1. Verfahren zur Herstellung von Di- und Polyaminen der Diphenylmethanreihe, bei dem man
a) Nitrobenzol und Methanol in Gegenwart eines Katalysators zu Anilin, Formaldehyd und Kohlenmonoxid umsetzt, und anschließend
b) das in Schritt a) hergestellte Anilin und Formaldehyd in Gegenwart eines sauren Katalysators zu Di- und Polyaminen der Diphenylmethanreihe umsetzt, und
c) das in Schritt a) hergestellte Kohlenmonoxid mit Chlor zu Phosgen umsetzt.

2. Verfahren nach Anspruch 1, bei dem man in Schritt a) einen Katalysator einsetzt, der ein oder mehrere Metalle, ausgewählt aus der Gruppe Pd, Pt, Rh, Ir, Ru, Fe, Co, Ni, Cu, Al, Mg, Zr, Zn, V, Cr, Mo, W, Pb, Lanthanoide, als katalytisch wirksame Komponenten in elementarer oder gebundener Form enthält.

3. Verfahren nach Anspruch 1 oder 2, bei dem Schritt a) in Gegenwart einer Base durchgeführt wird.

4. Verfahren nach einem der Ansprüche 1 bis 3, bei dem man aus dem in Schritt a) hergestellten Anilin und Formaldehyd das Anilin und/oder Formaldehyd teilweise entfernt.

5. Verfahren nach einem der Ansprüche 1 bis 4, bei dem man aus dem in Schritt a) hergestellten Anilin und Formaldehyd die Nebenprodukte Ameisensäure und/oder CO₂ und/oder Carbonate und/oder Ameisensäuremethylester und/oder N-Formylanilin und/oder N-Methylanilin teilweise oder vollständig abtrennt.

6. Verfahren nach einem der Ansprüche 1 bis 5, bei dem als saurer Katalysator in Schritt b) Salzsäure eingesetzt wird.

7. Verfahren nach Anspruch 1, bei dem man Anilin und Formaldehyd in Schritt a) im molaren Verhältnis von 0,9:1 bis 2,4:1 erhält.

8. Verfahren nach Anspruch 1, bei dem man Anilin und Kohlenmonoxid in Schritt a) im molaren Verhältnis von 0,5:1 bis 10:1 erhält.

9. Verfahren zur Herstellung von Di- und Polyisocyanaten der Diphenylmethanreihe, bei dem man
a) Nitrobenzol und Methanol in Gegenwart eines Katalysators zu Anilin, Formaldehyd und Kohlenmonoxid umsetzt, und anschließend
b) das in Schritt a) hergestellte Anilin und Formaldehyd in Gegenwart eines sauren Katalysators zu Di- und Polyaminen der Diphenylmethanreihe umsetzt, und
c) das in Schritt a) hergestellte Kohlenmonoxid mit Chlor zu Phosgen umsetzt, und
d) die in Schritt b) hergestellten Di- und Polyamine der Diphenylmethanreihe durch Phosgenierung zu den Di- und Polyisocyanaten der Diphenylmethanreihe umsetzt.

## Claims

1. Process for the production of di- and polyamines of the diphenylmethane series, in which
a) nitrobenzene and methanol are converted in the presence of a catalyst to aniline, formaldehyde and carbon monoxide, and then
b) the aniline and formaldehyde produced in step a) are converted in the presence of an acid catalyst to di- and polyamines of the diphenylmethane series, and
c) the carbon monoxide produced in step a) is converted with chlorine to phosgene.

2. Process according to claim 1, in which a catalyst is used in step a), which contains one or more metals, selected from the group Pd, Pt, Rh, Ir, Ru, Fe, Co, Ni, Cu, Al, Mg, Zr, Zn, V, Cr, Mo, W, Pb, lanthenoids, as catalytically-active components in elemental or bonded form.

3. Process according to claim 1 or 2, in which step a) is carried out in the presence of a base.

4. Process according to one of claims 1 to 3, in which the aniline and/or formaldehyde is partially removed from the aniline and formaldehyde produced in step a).

5. Process according to one of claims 1 to 4, in which the by-products formic acid and/or CO₂ and/or carbonates and/or formic acid methylester and/or N-formylaniline and/or N-methylaniline are partially or fully separated off from the aniline and formaldehyde produced in step a).

6. Process according to one of claims 1 to 5, in which hydrochloric acid is used as the acid catalyst in step b).

7. Process according to claim 1, in which aniline and formaldehyde are obtained in step a) in the molar ratio of 0.9:1 to 2.4:1.

8. Process according to claim 1, in which aniline and carbon monoxide are obtained in step a) in the molar ratio of 0.5:1 1 to 10:1.

9. Process for the production of di- and polyisocyanates of the diphenylmethane series, in which
a) nitrobenzene and methanol are converted in the presence of a catalyst to aniline, formaldehyde and carbon monoxide, and then
b) the aniline and formaldehyde produced in step a) are converted in the presence of an acid catalyst to di- and polyamines of the diphenylmethane series, and
c) the carbon monoxide produced in step a) is converted with chlorine to phosgene, and
d) the di- and polyamines of the diphenylmethane series produced in step b) are converted by phosgenation to the di- and polyisocyanates of the diphenylmethane series.

## Revendications

1. Procédé de préparation de di- et de polyamines de la série du diphénylméthane, dans lequel
a) on transforme du nitrobenzène et du méthanol, en présence d'un catalyseur, en aniline, en formaldéhyde et en monoxyde de carbone,
b) on transforme ensuite l'aniline et le formaldéhyde produits au cours de l'étape a), en présence d'un catalyseur acide, en di- et en polyamines de la série du diphénylméthane, et
c) on transforme le monoxyde de carbone produit au cours de l'étape a) avec du chlore en phosgène.

2. Procédé selon la revendication 1, dans lequel on utilise au cours de l'étape a) un catalyseur qui contient un ou plusieurs métaux sélectionnés parmi le groupe constitué des éléments suivants: Pd, Pt, Rh, Ir, Ru, Fe, Co, Ni, Cu, Al, Mg, Zr, Zn, V, Cr, Mo, W, Pb et lanthanides, en tant que composants possédant une activité catalytique, sous forme élémentaire ou liée.

3. Procédé selon la revendication 1 ou 2, dans lequel l'étape a) est réalisée en présence d'une base.

4. Procédé selon l'une des revendications 1 à 3, dans lequel on élimine partiellement l'aniline et/ou le formaldéhyde de l'aniline et du formaldéhyde produits au cours de l'étape a).

5. Procédé selon l'une des revendications 1 à 4, dans lequel on sépare partiellement ou complètement de l'aniline et du formaldéhyde produits au cours de l'étape a), les sous-produits acide formique et/ou CO₂ et/ou carbonates et/ou ester méthylique de l'acide formique et/ou N-formylaniline et/ou N-méthylaniline.

6. Procédé selon l'une des revendications 1 à 5, dans lequel on utilise de l'acide chlorhydrique comme catalyseur acide au cours de l'étape b).

7. Procédé selon la revendication 1, dans lequel on obtient l'aniline et le formaldéhyde au cours de l'étape a), dans un rapport molaire compris entre 0,9:1 et 2,4:1.

8. Procédé selon la revendication 1, dans lequel on obtient l'aniline et le monoxyde de carbone au cours de l'étape a), dans un rapport molaire compris entre 0,5:1 et 10:1.

9. Procédé de préparation de di- et de polyisocyanates de la série du diphénylméthane dans lequel
a) on transforme du nitrobenzène et du méthanol, en présence d'un catalyseur, en aniline, en formaldéhyde et en monoxyde de carbone,
b) on transforme ensuite l'aniline et le formaldéhyde produits au cours de l'étape a), en présence d'un catalyseur acide, en di- et en polyamines de la série du diphénylméthane,
c) on transforme le monoxyde de carbone produit au cours de l'étape a) avec du chlore en phosgène, et
d) on transforme les di- et les polyamines de la série du diphénylméthane produits au cours de l'étape b), par phosgénation en di- et en polyisocyanates de la série du diphénylméthane.
